(19) Europäisches Patentamt
European Patent Office
Office européen des brevets

(11) Numéro de publication: **0 136 210 B1**

# (12) FASCICULE DE BREVET EUROPEEN

(45) Date de publication du fascicule du brevet: **16.08.89**

(21) Numéro de dépôt: **84401621.2**

(22) Date de dépôt: **03.08.84**

(51) Int. Cl.⁴: **C 07 F 9/46**, C 07 F 9/65, C 07 F 15/00, C 07 C 45/49, C 07 C 2/46, C 07 C 143/46, C 07 C 29/136

(54) **Ligands phosphorés chiraux, leur procédé de fabrication et leur application à catalyse de réactions de synthèse énantiosélective.**

(30) Priorité: **05.08.83 FR 8312953**

(43) Date de publication de la demande: **03.04.85 Bulletin 85/14**

(45) Mention de la délivrance du brevet: **16.08.89 Bulletin 89/33**

(84) Etats contractants désignés: **AT BE CH DE FR GB IT LI LU NL SE**

(56) Documents cités:
**TETRAHEDRON LETTERS, vol. 23, no. 29, 1982, pages 2995-2996, Pergamon Press Ltd., GB; E. CESAROTTI et al.: "Asymmetric hydrogenation by chiral aminophosphine-phosphinite rhodium complexes"**
**CHEMISTRY LETTERS, no. 12, décembre 1981, pages 1691-1694, The Chemical Society of Japan, Tokyo, JP; K. OSAKADA et al.: "New chiral aminophosphines prepared from L-ornithine and catalytic asymmetric hydrogenation using their rhodium(I) complexes"**
**CHEMICAL ABSTRACTS, vol. 100, no. 23, 4 juin 1984, page 512, no. 191019d, Columbus, Ohio, USA; M. PETIT et al.: "Preparation of chiral aminophosphine-phosphinites. Applications to asymmetric homogeneous catalysis" & NOUV. J. CHIM. 1983, 7(10), 593-596**

(73) Titulaire: **NORSOLOR S.A., Tour Aurore Place des Reflets, F-92080 Paris la Défense Cédex 5 (FR)**

(72) Inventeur: **Petit, Michèle, 10 Clos des Courtilles Tranche 2, F-62152 Hardelot (FR)**
Inventeur: **Mortreux, André, 17 rue Gambetta, F-59510 Hem (FR)**
Inventeur: **Petit, Francis, 10 Clos des Courtilles Tranche 2, F-62152 Hardelot (FR)**
Inventeur: **Buono, Gérard, 171, Avenue des Caillots, Bat. A, F-13012 Marseille (FR)**
Inventeur: **Peiffer, Gilbert, 9, Avenue Paul Carrère, F-13012 Marseille (FR)**

(74) Mandataire: **Dubost, Thierry, c/o NORSOLOR Service Propriété Industrielle B.P. 57, F-62670 Mazingarbe (FR)**

EP 0 136 210 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une famille de nouveaux ligands phosphorés chiraux, un procédé pour leur fabrication et de nouveaux complexes de métaux de transition comportant lesdits ligands, utiles comme catalyseurs dans des réactions de synthèse énantiosélective de composés organiques.

L'intérêt industriel de la synthèse de substances optiquement actives catalysée par des complexes de métaux de transition contenant des ligands chiraux est bien connu. De telles réactions catalytiques en vue de la création d'au moins un centre d'asymétrie dans une molécule organique présentent toutefois une grande difficulté et ne sont maîtrisées industriellement que dans des cas très particuliers. Ainsi il est connu (brevets américains n° 4 005 127, 4 142 992 et 4 220 590) de préparer la (dihydroxy-3,4 phényl)-3 alanine (L-DOPA) par hydrogénation de l'acide méthoxy-3, acétoxy-4 acétamidocinnamique au moyen d'un catalyseur homogène à base de rhodium coordiné par un ligand phosphoré, suivie d'hydrolyse. Toutefois la synthèse de ce ligand nécessite une série de cinq étapes parmi lesquelles une séparation de diastéréoisomères qui augmente de manière conséquente le prix de revient global de l'opération (B.D. Vineyrard, W.S. Knowles et M.J. Sabacky, Journal of Molecular Catalysis, 19 (1983) p. 161). On rappelle que la L-DOPA est utilisée comme principe actif pour le traitement de la maladie de Parkinson. E. Cesarotti, A. Chiesa et G. D'Alfonso ont décrit dans Tetrahedron Letters, vol. 23, n° 29 (1982), pages 2995–6, l'hydrogénation assymétrique, à 20 °C et sous pression atmosphérique, des acides α-N-acétaminoacrylique, α-N-acétaminocinnamique et itaconique en présence d'un complexe cationique de formule Rh(COD)L $ClO_4$ dans laquelle COD désigne le cyclooctadiène et L désigne le ligand N-(diphénylphosphino)-2-diphénylphosphinoxyméthyl pyrrolidine.

Le but de la présente invention consiste en des ligands phosphorés chiraux qui puissent être fabriqués selon un procédé simple et peu onéreux, capables d'entrer dans la constitution de complexes de métaux de transition utiles comme catalyseurs dans différentes réactions de synthèse énantiosélective de composés organiques.

Un premier objet de la présente invention est une famille de nouveaux ligands phosphorés chiraux, comprenant au moins un radical amine et au moins un radical dihydrocarbylphosphinoxy de formule W = $OP(R)_2$ dans laquelle R est un radical hydrocarboné choisi parmi les radicaux alkyles aryles et cycloalkyles, caractérisés en ce qu'ils sont choisis parmi:

– ceux de formule

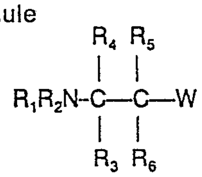

(I)

dans laquelle

– $R_1$ et $R_2$ sont choisis parmi l'atome hydrogène et les radicaux hydrocarbones,
– $R_3$ et $R_4$, nécessairement différents, sont choisis parmi l'atome d'hydrogène et les radicaux hydrocarbonés éventuellement porteurs d'au moins une fonction choisie parmi les fonctions alcool, thiol, thiother, amine, imine, acide, ester, amide et ether, et
– $R_5$ et $R_6$ sont choisis parmi l'atome d'hydrogène et les radicaux hydrocarbonés éventuellement fonctionnalisés et les composés suivants:
– le (1R,2S) phényl-1-(oxydiphénylphosphino)-1-(N-méthyl-N-diphénylphosphino amino)-2-méthyl-2-éthane,
– le (1R,2R) phényl-1-(oxydiphénylphosphino)-1-(N-méthyl-N-diphénylphosphino-amino)-2-méthyl-2-éthane,
– le (2S,4R)-N-diphénylphosphino-carbethoxy-2-(oxydiphényl-phosphino)4-pyrrolidine,
– le N-dicyclohexylphosphino-(oxyméthylène dicyclohexylphosphino)-2-pyrrolidine,
– le (S)N-diphénylphosphino-(oxyméthylène diphénylphosphino)-2-pyrrolidine,
– le (S)(oxidiphénylphosphino)-1-(N-méthyl-N-diphénylphosphino-amino)-2-phényl-2-éthane,
– le (S)(oxydiphénylphosphino)-1-(N-méthyl-N-diphénylphosphino-amino)-2-méthyl-2-éthane,
– le (S)(oxydiphénylphosphino)-1-(N-méthyl-N-diphénylphosphino-amino)-2-isopropyl-2-éthane,
– le (S)(oxydiphénylphosphino)-1-(N-méthyl-N-diphénylphosphino-amino)-2-isobutyl-2-éthane,
– le (S)(oxydiphénylphosphino)-1-(N-méthyl-N-diphénylphosphino)-1-(N-méthyl-N-diphénylphosphinoamino)-2-benzyl-2-éthane,
– le (S)-bis(oxydiphénylphosphino)-1,5-(N-méthyl-N-diphénylphosphinoamino)-2-pentane,
– le (S)-bis(oxydiphénylphosphino)-1,4-(N-méthyl-N-diphénylphosphinoamino)-2-butane,
– le (2S,3R)-bis(oxydiphénylphosphino)-1,3-(N-méthyl-N-diphénylphosphinoamino)-2-butane, et
– le (S)N,N'-bisdiphénylphosphino-(méthylamino)-2-(oxydiphénylphosphino)-1-(3-indolyl)-3-propane.

Ces composés sont des ligands phosphorés chiraux, comprenant au moins un radical amine et au moins un radical dihydrocarbylphosphinoxy de formule W = $OP(R)_2$ décrit ci-dessus. Ces ligands répondent essentiellement à l'une des formules générales suivantes:

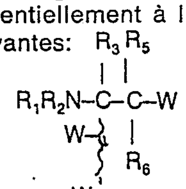

(II),

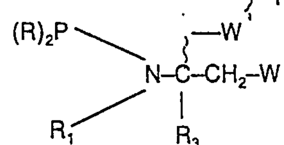

(III) et

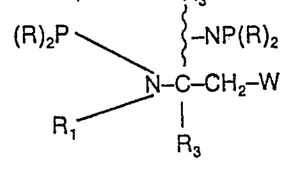

(IV),

La famille des ligands selon l'invention comprend trois sous-familles selon le nombre de radicaux dihydrocarbylphosphine présents et selon leur place dans la molécule:

1) la sous-famille des chélates essentiellement monodentés de formule (I), que nous désignerons par la suite par le terme «aminophosphinites».

2) la sous-famille des chélates essentiellement bidentés répondant à la formule (II), que nous désignerons par la suite par le terme «aminodiphosphinites».

3) la sous-famille des chélates essentiellement tridentés comprend d'une part des «aminophosphinediphosphinites» répondant à la formule (III) et d'autre part des «diaminophosphine-phosphinites» répondant à la formule (IV).

Des exemples de radical R sont notamment les radicaux méthyle, éthyle, isopropyle, tertiobutyle, cyclohexyle et phényle. L'exemple le plus fréquent de radical hydrocarboné $R_1$ ou $R_2$ est le radical méthyle.

Des exemples de radicaux $R_3$ ou $R_4$ sont notamment les radicaux méthyle, isopropyle, isobutyle, isoamyle, n-hydroxyéthyle, iso-hydroxyéthyle, n-aminobutyle, méthylène-4 imidazolyle, N-n-propylguanidyle, éthanoyle, acétamidoyle, n-propionamidoyle, benzyle, p-hydroxybenzyle, méthylène-3 indolyle, méthanethioyle.

Un second objet de la présente invention est un procédé de fabrication des nouveaux ligands phosphorés chiraux décrits ci-dessus. Le procédé selon l'invention consiste à faire réagir dans un solvant hydrocarboné, à une température comprise entre −50 °C et 80 °C et sous atmosphère de gaz inerte un aminoalcool optiquement actif de formule générale

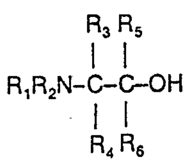

(V)

avec au moins un composé de formule $P(R)_2$ Y dans laquelle Y est choisi parmi les atomes d'halogène et les radicaux amines, ledit composé étant dans un rapport molaire, relativement à l'aminoalcool, supérieur ou égal au nombre de groupes $P(R)_2$ à introduire dans la molécule chirale.

Par solvant hydrocarboné au sens de la présente invention on entend par exemple le benzène, le toluène, etc.

Pour la synthèse des ligands de formules (I) et (II), on préfère utiliser un composé dans lequel Y est un radical amine.

Pour la synthèse des ligands de formules (III) et (IV), on préfère utiliser un composé dans lequel Y est un atome d'halogène. Pour faciliter l'élimination de l'hydrure d'halogène formé par la réaction, on peut alors avantageusement conduire celle-ci en présence d'un excès d'amine tertiaire, telle que par exemple la triéthylamine, qui précipitera sous forme d'halogènohydrate. Le ligand phosphoré chiral est isolé successivement par filtration du précipité suivie d'évaporation du solvant hydrocarboné sous vide. Il se présente généralement sous forme d'une huile visqueuse. Les aminoalcools chiraux de formule (V) sont le plus souvent des produits soit commerciaux soit facilement accessibles par réduction d'aminoacides naturels (ou de leurs esters méthyliques formylés). Lorsque le radical $R_3$ ou $R_4$ de l'aminoalcool de formule (V) est porteur d'une fonction, celle-ci peut être introduite par une réaction de fonctionnalisation bien connue, par exemple la fonction ester sera introduite par estérification de la fonction acide correspondante (cas des carbalcoxyhydroxyprolines). A titre d'exemples on peut citer le prolinol, l'hydroxyproline, les éphédrines et les aminoalcools N-méthylés dérivés des aminoacides naturels suivants: glycine, phénylglycine, phénylalanine, leucine, sérine, thréonine, histidine, lysine, arginine, isoleucine, valine, alanine, tyrosine, tryptophane, méthionine, cystéine, glutamine, asparagine, acide aspartique, acide glutamique, cystine.

La présente invention concerne aussi des applications particulières de ligands de la famille des «aminophosphine-phosphinites» de formule:

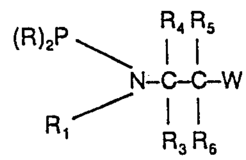

dans laquelle R, $R_1$, $R_3$, $R_4$, $R_5$ et $R_6$ ont la même signification que précédemment. Certains des ligands de cette famille n'ont pas été décrits dans l'art antérieur mentionné précédemment.

Les ligands phosphorés chiraux selon l'invention peuvent être identifiés notamment à l'aide de leurs spectres de résonance magnétique nucléaire du proton, du carbone 13 et du phosphore 31, ainsi que de leur pouvoir rotatoire spécifique. Le tableau I ci-après résume les données d'identification de certains aminophosphine-phosphinites, selon l'aminoalcool ou l'aminoacide dont ils sont issus.

Le tableau II ci-après résume les données d'identifiction de certains aminophosphinites, dans lesquels R est le radical phényle, selon l'aminoalcool ou l'aminoacide dont ils sont issus.

Le tableau III ci-après résume les données d'identification de certains aminophosphinediphosphinites et, en ce qui concerne le ligand issu du tryptophane, d'un diaminophosphine-phosphinite dans lesquels R est le radical phényle.

Dans ces tableaux $[\alpha]_D^{25}$ désigne le pouvoir rotatoire spécifique à 25 °C exprimé en degrés pour la raie D et δ désigne le déplacement chimique exprimé en ppm par rapport à l'acide phosphorique (cas du phosphore 31) ou au tétraméthylsilane (cas du proton et du carbone 13). Les notations (m), (dd), (d) et (s) désignent respectivement un multiplet, un double doublet, un doublet et un singulet.

Un troisième objet de la présente invention concerne l'application des nouveaux ligands phos-

Tableau I

| Ligand n° | Précurseur aminoalcool ou aminoacide | Nom du ligand | $\delta^{31}_{P}$ | $\delta^{13}_{C}$ | $\delta^{1}_{H}$ | $\alpha^{25}_{D}$ |
|---|---|---|---|---|---|---|
| 1 | Ephédrine érythro(+) | (1R,2S) phényl-1-(oxydiphénylphosphino)-1-(N-méthyl-N diphénylphosphino amino)-2-méthyl-2-éthane | 111,1<br>63,8 | 129–134 (m)<br>86,9 (dd)<br>65,8 (dd)<br>31,8 (d)<br>17,1 (d) | 7–8<br>4,8<br>4,0<br>2,2<br>1,3 | −60,5 |
| 2 | Ephédrine thréo (−) | (1R,2R) phényl-1-(oxydiphénylphosphino)-1-(N-méthyl-N diphénylphosphino amino)-2-méthyl-2-éthane | 110,5<br>63,7 | 129–130 (m)<br>86,8 (dd)<br>66,2 (dd)<br>31,0 (d)<br>16,6 (d) | 7–8<br>4,95<br>4,0<br>2,3<br>1,15 | −16,1 |
| 3 | Carbéthoxyhydroxyproline | (2S, 4R)-N-diphénylphosphino-carbethoxy-2-(oxydiphényl-phosphino)-4-pyrrolidine | 108,4<br>46,3 | | | −96,2 |
| 4(a) | Prolinol | N-dicyclohexylphosphino-(oxyméthylène dicyclohexylphosphino)-2-pyrrolidine | 146,5<br>53,9 | 74,9 (m)<br>64,4 (dd)<br>47,2 (d)<br>23–30 (m)<br>24,4 (s) | | −37,4 |
| 5 | Prolinol (+) | (S)N-diphénylphosphino-(oxyméthylène diphénylphosphino)-2-pyrrolidine | 113,5<br>45,6 | 128–133 (m)<br>73,1 (dd)<br>63,8 (dd)<br>47,2 (d)<br>29,6 (d)<br>25,4 (s) | 7,1–8<br>3,3–4<br>3,05<br>2,07<br>1,5–2,1 | +22,6<br>(b) |
| 6 | Phényglycine D (−) | (S)(oxydiphénylphosphino)-1-(N-méthyl-N-diphénylphosphino amino)-2-phényl-2-éthane | 114,9<br>59,3 | 127–133 (m)<br>69,5 (dd)<br>68,7 (dd)<br>33,4 (d) | 7,2<br>4,3<br>4,2<br>2,3 | − 3,7 |
| 7 | Alanine | (S)(oxydiphénylphosphino)-1-(N-méthyl-N-diphénylphosphino amino)-2-methyl-2-éthane | 113,2<br>58,1 | 125–136 (m)<br>72,6 (dd)<br>61,0 (dd)<br>32,5 (d)<br>16,8 (d) | | +23,7 |

(a) dans ce ligand, R est le radical cyclohexyle
(b) valeur mesurée pour la raie à 402 nm au lieu de la raie D.

Tableau I (suite)

| Ligand n° | Précurseur aminoalcool ou aminoacide | Nom du ligand | $\delta_P^{31}$ | $\delta_C^{13}$ | $\delta_D^{25}$ |
|---|---|---|---|---|---|
| 8 | Valine | (S)(oxydiphénylphosphino)-1-(N-méthyl-N-diphénylphosphino amino)-2-isopropyl-2-éthane | 114,3<br>62,0 | 127 – 134 (m)<br>73,1 (dd)<br>71,1 (dd)<br>33,5 (d)<br>29,0<br>21,35<br>21,3 | + 4,4 |
| 9 | Leucine | (S)(oxydiphénylphosphino)-1-(N-méthyl-N-diphénylphosphino amino)-2-isobutyl-2-éthane | 113,8<br>59,5 | 126–142 (m)<br>72,2 (dd)<br>64,0 (dd)<br>39,9 (d)<br>32,2 (d)<br>24,6<br>23,5<br>21,9 | +15,4 |
| 10 | Phénylalanine | (S)(oxydiphénylphosphino)-1-(N-méthyl-N diphénylphosphino amino)-2-benzyl-2 éthane | 114,2<br>60,0 | 126–140 (m)<br>71,7 (dd)<br>66,8 (dd)<br>37,5 (d)<br>32,7 (d) | + 1,1 |

Tableau II

| Ligand n° | Précurseur aminoalcool ou aminoacide | Nom du ligand | $\delta_P^{31}$ | $\delta_C^{12}$ | $\delta_H^1$ | $\alpha_D^{25}$ |
|---|---|---|---|---|---|---|
| 11 | Ephédrine érythro (+) | (1R,2S)phényl-1-(oxydiphénylphosphino)-1-(N-méthylamino)-2-méthyl-2-éthane | 112,3 | 127 – 132 (m)<br>84,5 (d)<br>61,1 (d)<br>31,8 (s)<br>15,2 (s) | 0,92–1,03 (d) (3H)<br>1,59 (s) (1H)<br>2,23 (m) (3H)<br>2,82 (m) (1H)<br>4,78–5,01 (m) (1H)<br>7,27 (m) (10H) | |
| 12 | Valine | (S)(oxydiphénylphosphino)-1-(N-méthylamino)-2-isopropyl-2-éthane | 113,7 | 127–135 (m)<br>69,2 (d)<br>65,9 (d)<br>34,5<br>29,3<br>18,9<br>18,8 | | +25,9 |
| 13 | Prolinol | (S)(oxyméthylene diphénylphosphino)-2-pyrrolidine | 113,1 | | 1,69 (m) (5H)<br>2,85 (m) (2H)<br>3,20 (m) (1H)<br>3,64–3,87 (m) (2H)<br>7,4 (m) (10H) | |
| 14 | Glycine | (S)(oxydiphénylphosphino)-1-(N-méthylamino)-2-éthane | 113,5 | | 1,31 (s) (1H)<br>2,30 (s) (3H)<br>2,73 (t) (2H)<br>3,80–3,97 (t–d) (2H)<br>7,14–7,48 (m) (10H) | |

Tableau III

| Ligand n° | Précurseur aminoalcool ou aminoacide | Nom du ligand | $\delta^{31}_{P}$ | $\alpha^{25}_{D}$ |
|---|---|---|---|---|
| 15 | Acide glutamique | (S)-bis(oxydyphénylphosphino)-1,5-(N-méthyl-N-diphénylphosphinoamino)-2-pentane | 113,8 (s)<br>111,0 (s)<br>58,6 (s) | + 1,4 |
| 16 | Asparagine | (S)-bis(oxydiphénylphosphino)-1,4-(N-méthyl-N-diphénylphosphinoamino)-2-butane | 114,0 (s)<br>111,7 (s)<br>57,5 (s) | − 11,5 |
| 17 | Thréonine | (2S,3R)-bis(oxydiphénylphosphino)-1,3-(N-méthyl-N-diphénylphosphinoamino)-2-butane | 114,0 (s)<br>111,5 (s)<br>64,6 (s) | + 13,8 |
| 18 | Tryptophane | (S)N,N'-bisdiphénylphosphino-(méthylamino)-2-(oxydiphénylphosphino)-1-(3-indolyl)-3-propane | 113,5 (s)<br>59,1 (s)<br>16,8 (s) | − 5,5 |

phorés chiraux décrits précédemment à des réactions de synthèse énantiosélective de composés organiques. Cette application consiste à faire réagir au moins un composé organique ne possédant pas de centre d'asymétrie avec (A) d'une part au moins un complexe de métal de transition de formule MZq dans laquelle M est un métal du groupe VIII de la Classification Périodique, q est le degré de coordination du métal M et Z est un atome ou une molécule capable de complexer le métal M et (B) d'autre part au moins un ligand phosphoré chiral L décrit précédemment. Le cas échéant ladite réaction peut être effectuée en présence de (C) au moins un agent capable de capter au moins un ligand Z du constituant (A). Cet agent (C) peut être soit un acide possédant un anion $A^-$ peu coordinant et stériquement encombré ou un sel métallique d'un tel acide, soit une quantité d'électricité mise en jeu par électrolysé à potentiel cathodique imposé. Par anion $A^-$ peu coordinant et stériquement encombré on entend notamment les anions perchlorate, tétrafluoro- et tétraphénylborate, hexafluorophosphate. Les sels métalliques utilisables sont principalement ceux d'argent et de thallium.

D'autre part ladite réaction de synthèse énantiosélective peut être effectuée, le cas échéant, en présence de (D) au moins un activateur choisi parmi les dérivés aluminiques de formule $AlR_nX_{3-n}$, dans laquelle $0 \leq n \leq 3$, X est un atome d'halogène et R est un radical alkyle ayant de 1 à 12 atomes de carbone. Les constituants (A) et (B) sont généralement dans un rapport molaire (B)/(A) compris entre 1 et 10. Les constituants (A) et (D) sont généralement dans un rapport molaire (D)/(A) compris entre 0,1 et 10. Le constituant (A) et l'agent (C) sont généralement dans un rapport molaire (C)/(A) inférieur ou égal à q, lorsque (C) est un acide ou sel.

Parmi les métaux M couramment utilisables on peut citer le fer, le nickel, le cobalt, le rhodium, le ruthénium, l'iridium, le palladium, le platine. Parmi les atomes ou molécules Z couramment utilisables on peut citer le monoxyde de carbone, les halogènes, l'éthylène, le norbornadiène, le cyclooctadiène, l'acétylacétone. Enfin le degré de coordination q peut être couramment compris entre 2 et 6 inclusivement selon le métal et/ou les ligands utilisés.

Tout composé organique ne possédant pas de centre d'asymétrie peut être soumis à une réaction de synthèse énantiosélective selon l'invention. Il en va ainsi de certaines oléfines, de certaines cétones, de certains diènes, éventuellement fonctionnalisés. Des exemples de réactions selon l'invention sont les réactions bien connues suivantes:

– hydrogénation et hydroformylation de substrats organiques insaturés tels que les oléfines éventuellement fonctionnalisées en α de la double liaison, en particulier les déhydroaminoacides ou leurs dérivés N-acétylés tels que les acides acétamidocarboxyliques; les réactions d'hydrogénation sont généralement effectuées à une température comprise entre 0 et 200 °C et sous une pression

comprise entre 1 et 200 bars; les réactions d'hydroformylation sont généralement effectuées à une température comprise entre 15 et 300 °C et sous une pression comprise entre 1 et 350 bars; ces réactions peuvent être appliquées industriellement notamment à la synthèse d'édulcorants non nutritifs comme l'aspartame et le (R)6-méthyltryptophane.

– réduction de cétones et d'imines prochirales par des organosilanes, en particulier des monohydrosilanes et, de préférence, des dihydrosilanes (réaction d'hydrosilylation).

– cyclodimérisation et dimérisation des diènes conjugués, et codimérisation de l'éthylène et d'un diène conjugué; ces réactions sont généralement effectuées à une température comprise entre −30 et +100 °C et sous une pression inférieure à 100 bars.

Un quatrième objet de la présente invention concerne, à titre de produits nouveaux utilisés comme intermédiaires dans l'application décrite précédemment, des complexes de métaux de transition choisis parmi d'une part ceux de formule $MZ_{q-r}L_r$, et d'autre part ceux de formules $(MZ_{q-r}L_r)^+A^-$, formules dans lesquelles M, Z, A et q ont les mêmes significations que précédemment, L désigne le ligand phosphoré chiral et r est égal à 1 ou 2. Ces complexes se forment, dans le cadre de ladite application, par réaction entre le complexe de formule MZq d'une part et le ligand L d'autre part, le cas échéant en présence d'au moins un acide ou sel possédant un anion $A^-$.

Ces complexes de métaux de transition peuvent être identifiés notamment à l'aide de leur spectre de résonance magnétique nucléaire du phosphore 31. Le tableau IV ci-après résume les données d'identification de certains complexes de formule

$$[Rh\ (Norbornadiène)\ (L)]^+\ CLO_4^-$$

selon la nature du ligand L.

Tableau IV

| Ligand n° | 1 | 2 | 3 | 5 | 6 |
|---|---|---|---|---|---|
| $\delta_P^{31}$ | 120,9 | 122,4 | 110,4 | 122,8 | 110,7 |
| | 90,8 | 95,3 | 96,8 | 71,5 | 85,5 |
| Ligand n° | 7 | 8 | 9 | 10 | 18 |
| $\delta_P^{31}$ | 119,5 | 119,5 | 118,8 | 116,7 | 118,0 |
| | 87,3 | 88,8 | 87,8 | 88,5 | 88,3 |
| | | | | | 33,6 |

Les exemples suivants sont donnés à titre illustratif et non limitatif de la présente invention.

EXEMPLE 1

On fait réagir dans le benzène sec, à une température de +5 °C et sous atmosphère d'azote, 2 moles de chlorodiphénylphosphine avec 1 mole d'éphédrine (méthylamino-2 phényl-1 propanol-1) érythro (+) (rendue anhydre par distillation azéotropique de l'eau par le benzène) en présence d'un excès de triéthylamine. On filtre le précipité de chlorhydrate de triéthylammonium qui se forme, puis on évapore le benzène sous vide. Une recristallisation effectuée dans le benzène permet d'éliminer un sous-produit identifié comme la méthylamino bis (diphénylphosphine): l'analyse par résonance magnétique nucléaire du proton révèle en effet des déplacement chimiques δ à 7,6 et 3,5 ppm par rapport au tétraméthylsilane. On obtient alors 0,8 mole du ligand n° 1 (voir tableau I), de pureté chimique évaluée à 95%.

EXEMPLE 2

On répète la méthode de synthèse décrite à l'exemple 1 en remplaçant l'éphédrine érythro (+) respectivement par:

– l'éphédrine thréo (−) en vue d'obtenir le ligand n° 2.

– le S (+) prolinol en vue d'obtenir le ligand n° 5.

– le R (−) méthylphénylglycinol en vue d'obtenir le ligand n° 6.

– le N-méthyl amino-2 propanol en vue d'obtenir le ligand n° 7.

– l'alcool N-méthylé dérivé de l'acide α-amino-isovalérique (valine) en vue d'obtenir le ligand n° 8.

– l'alcool N-méthylé dérivé de l'acide amino-2 méthyl-4 n-valérique (leucine) en vue d'obtenir le ligand n° 9.

– le N-méthyl amino-2 phényl-3 propanol en vue d'obtenir le ligand n° 10.

– le carbéthoxyhydroxyprolinol en vue d'obtenir le ligand n° 3.

On obtient ces ligands avec un rendement identique ou similaire à celui de l'exemple 1.

Pour mémoire, on rappelle que le S (+) prolinol peut être aisément obtenu par réduction de l'acide pyrrolidine-2 carboxylique (proline) au moyen de l'hydrure double de lithium et aluminium $LiAlH_4$ en suspension dans le tétrahydrofurane. De même les autres aminoalcools sont préparés à partir des aminoacides naturels correspondants par monoformylation de la fonction amine, estérification de la fonction acide, puis réduction par $LiAlH_4$.

EXEMPLE 3

On répète la méthode de synthèse décrite à l'exemple 1 en remplaçant d'une part l'éphédrine érythro (+) par le S (+) prolinol et d'autre part la chlorodiphénylphosphine par la chlorodicyclo-

hexylphosphine. On obtient alors, avec le même rendement, le ligand n° 4 (voir tableau I).

EXEMPLE 4

On fait réagir dans le benzène sec sous atmosphère d'azote 1 mole de diméthylaminophénylphosphine avec 1 mole d'éphédrine érythro (+) (rendue anhydre par distillation azéotropique de l'eau par le benzène). Après dégagement de la diméthylamine, on évapore le benzène sous vide et l'on récupère 0,8 mole du ligand n° 11 (voir tableau II) de pureté chimique évaluée à 90%.

EXEMPLE 5

On répète la méthode de synthèse décrite à l'exemple 4 en remplaçant l'éphédrine érythro (+) successivement par:

- l'alcool N-méthylé dérivé de l'acide α-aminoisovalérique (valine) pour obtenir le ligand n° 12 (voir tableau II).
- l'alcool N-diméthylé dérivé de la valine pour obtenir le (S)(oxydiphénylphosphino)-1-(N-diméthylamino)-2-isopropyl-2 éthane identifié comme suit:

$\delta_P^{31} = 112,7$ (s)

On obtient ces ligands avec un rendement identique ou similaire à celui de l'exemple 4.

EXEMPLE 6

On fait réagir dans le benzène sec sous atmosphère d'azote 3 moles de chlorodiphénylphosphine avec 1 mole de l'alcool N-méthylé dérivé de l'acide glutamique en présence d'un excès de triéthylamine. On filtre le précipité de chlorhydrate de triéthylammonium qui se forme, puis on évapore le benzène sous vide. On obtient alors 0,8 mole du ligand n° 15 (voir tableau III) de pureté chimique évaluée à 85%.

EXEMPLE 7

On répète la méthode de synthèse décrite à l'exemple 6 en utilisant comme aminoalcools les alcools N-méthylés dérivés de:

- l'acide aminosuccinique (asparagine) pour obtenir le ligand n° 16.
- l'acide amino-2 hydroxy-3 butyrique (thréonine) pour obtenir le ligand n° 17.
- l'acide amino-2 (3-indolyl)-3 propionique (tryptophane) pour obtenir le ligand n° 18.

On obtient ces ligands avec un rendement identique ou similaire à celui de l'exemple 6.

EXEMPLE 8

On fait réagir, dans le benzène sec et à une température de 20 °C, 1 mole du ligand n° 1 (ci-après désigné L) et 0,5 mole de dichlorotétracarbonyldirhodium. On évapore le solvant puis, après lavage par un ether de pétrole et séchage sous vide, on récupère 0,8 mole d'un complexe de formule RhCl(CO)L. Ce complexe est identifié par son spectre de résonance magnétique nucléaire du phosphore 31, qui révèle des déplacement chimiques à 90,1 ppm(dd), 99,7 ppm(dd), 111,6 ppm(dd) et 128,7 ppm(dd).

EXEMPLE 9 – hydroformylation asymétrique

A 0,1 millimole du complexe de l'exemple 8 sont ajoutés successivement 10 $cm^3$ de benzène anhydre et 40 $cm^3$ de carbonate de propylène fraichement distillé. La solution est réalisée sous azote et est introduite sous gaz inerte dans un autoclave électrochimique de 320 $cm^3$ équipe de trois électrodes: une électrode de platine (cathode), une électrode de fer (anode) et une électrode de référence permettant une jonction liquide entre l'intérieur de l'autoclave et la pression atmosphérique extérieure. Cette dernière électrode est constituée par un tube percé en son centre dans lequel on introduit une mèche d'amiante. Celle ci s'imbibe du liquide solvant et un système de presse-étoupe permet de comprimer la mèche et de contrecarrer la pression intérieure, tout en permettant le passage d'un léger débit de liquide pour effectuer la jonction avec une électrode au calomel saturé située à l'extérieur de l'autoclave. Ce montage à trois électrodes est nécessaire pour effectuer une réduction à potentiel cathodique imposé, à l'aide d'un potentiostat. On ajoute ensuite 4,5 g de styrène fraichement distillé. Le réacteur est alors chargé d'un mélange $CO/H_2$ (1/1) à la température de 25 °C sous 10 atmosphères. On électrolyse la solution au potentiel cathodique de réduction de −0,9 volts, jusqu'à ce que l'intensité du courant devienne inférieure à 5% de sa valeur maximale, ce qui correspond à environ 1 Faraday par atome gramme de rhodium (temps de réduction = environ 4 heures). La chromatographie en phase gazeuse permet de déterminer un taux de conversion de 45% molaire du styrène en un mélange constitué de 93% de phényl-2 propanal et de 7% de phényl-3 propanal. Le benzène est éliminé du mélange réactionnel par évaporation sous vide, puis on effectue une rectification sous vide des produits. On obtient ainsi une solution d'aldéhydes dans le carbonate de propylène, débarassée de la présence du complexe métallique, sur laquelle on effectue directement la mesure du pouvoir rotatoire: le rendement optique est de 25% en aldéhyde de configuration S.

EXEMPLE 10 – Cyclodimérisation asymétrique

Dans un tube en verre on introduit successivement 687 mg (2,5 millimole) de bis(cyclooctadiène-1,5) nickel puis 947,5 mg (2,5 millimole) du ligand n° 9.

On solubilise ces produits dans 2 g d'heptane et environ 15 $cm^3$ de toluène, de manière à obtenir une concentration en catalyseur de 0,1 mole par litre après addition de 6,75 g (0,13 mole) de butadiène-1,3 par bullage dans la solution maintenue à 0 °C. Le mélange réactionnel est ensuite maintenu sous agitation à la température de 40 °C pendant 24 heures. La pression réactionnelle atteint environ 5 bars. En fin de réaction le catalysat est filtré sur silice pour éliminer le catalyseur et la solution limpide obtenue est distillée sur colonne pour séparer le vinyl-4 cyclohexène de son isomère cyclooctadiène-1,5, libéré à partir du catalyseur initial et formé au cours de la réaction. On obtient en effet, avec un rendement chimique de

85%, un mélange constitué essentiellement de 40% de cyclooctadiène-1,5 et 52% de vinyl-4 cyclohexène. Le vinyl-4 cyclohexène est obtenu avec une pureté optique de 25%.

EXEMPLE 11 – hydrogénation asymétrique

Dans un tube de Schlenk et sous azote on dissout 0,1 millimole de complexe de Cramer $Rh_2Cl_2(C_2H_4)_4$ dans 8 cm$^3$ d'éthanol à 95%, on ajoute à l'aide d'un tube de transfert une solution de 0,2 millimole du ligand n° 3 dans 12 cm$^3$ d'éthanol à 95% et on agite pendant 15 minutes sous azote à 20 °C. Le complexe ainsi obtenu, de formule RhLCl, est transféré sous hydrogène dans un réacteur préalablement purgé, contenant 0,01 mole d'acide acétamidoacrylique dissous dans 40 cm$^3$ d'éthanol à 95%. On introduit de l'hydrogène dans le réacteur et la réaction, effectuée à 20 °C sous pression atmosphérique, est suivie par lecture du volume d'hydrogène absorbé. Après 1 heure de réaction, l'éthanol est évaporé, le résidu est repris à l'eau chaude puis on filtre pour séparer le complexe insoluble. L'eau est évaporée et on récupère alors, avec un rendement chimique de 98%, la N-acétylalanine. Son pouvoir rotatoire spécifique, mesuré sur une solution aqueuse de concentration 2 g/100 cm$^3$, $[\alpha]_D^{25} = +48,9°$, correspond à un rendement optique de 73,5%.

EXEMPLE 12

On répète les opérations décrites à l'exemple 11 en remplaçant le ligand n° 3 par le ligand n° 4. On récupère avec le même rendement chimique la N-acétylalanine dont le pouvoir rotatoire spécifique $[\alpha]_D^{25} = +48,5°$ correspond à un rendement optique de 73%.

EXEMPLE 13 – hydrosilylation asymétrique

Dans un tube de Schlenk et sous azote, on dissout 0,2 millimole de complexe de Cramer $Rh_2Cl_2(C_2H_4)_4$ dans 10 cm$^3$ de benzène, on ajoute à l'aide d'un tube de transfert une solution de 0,4 millimole du ligand de n° 3 dans 10 cm$^3$ de benzène et on agite pendant 15 minutes sous azote à 20 °C. Le complexe ainsi obtenu, de forme RhLCl, est transféré sous azote dans un réacteur préalablement purgé, contenant 2,34 cm$^3$ d'acétophénone (0,02 mole), 4,1 cm$^3$ d'α-naphtyl phénylsilane (0,02 mole) et 30 cm$^3$ de benzène. La réaction est poursuivie, à 20 °C et sous pression atmosphérique, pendant 4 heures. Le benzène est ensuite évaporé sous vide. Le résidu est dissous dans 60 ml d'acétone contenant 12 ml d'une solution aqueuse d'acide chlorhydrique 10% et on agite pendant 2 heures. On extrait la phase organique à l'éther, on neutralise par une solution de carbonate de sodium 5%, puis par de l'eau distillée. La phase éthérée est ensuite séchée sur $MgSO_4$, filtrée et évaporée sous vide. Après distillation on récupère, avec un rendement chimique de 96%, le phényl-1 éthanol. Son pouvoir rotatoire spécifique $[\alpha_D^{25} = -22,4°$ correspond à un rendement optique de 42,6%.

EXEMPLE 14

On répète les opérations décrites à l'exemple 13 en remplaçant le ligand n° 3 par le ligand n° 8 et l'α-naphtyl phénylsilane par le diphénylsilane. On obtient également le phényl-1 éthanol avec un rendement chimique de 96% et un rendement optique de 26%.

EXEMPLE 15 – Codimérisation asymétrique

Dans un autoclave de 300 cm$^3$ préalablement purgé à l'azote on introduit successivement 110 mg (0,4 millimole) de bis(cyclooctadiène-1,5 nickel, 33 cm$^3$ de toluène anhydre, 2 g d'heptane, 220 mg (0,4 millimole) du ligand n° 17, 0,2 g (1,6 millimole) de chlorure de diéthylaluminium, puis 4 g (0,05 mole) de cyclohexadiène-1,3. L'autoclave est alors fermé puis chargé en éthylène jusqu'à atteindre une quantité équimolaire à celle du diène. La réaction est alors poursuivie sous agitation, à −30 °C et sous une pression de 6 bars, pendant 15 minutes. Après rectification on obtient, avec un rendement chimique de 80%, du (S)vinyl-3 cyclohexène pur à 97% et possédant un pouvoir rotatoire spécifique $[\alpha]_D^{25} = 245°$, soit un rendement optique de 92,3%.

EXEMPLE 16 – synthèse de $[Rh(Norbornadiène)L]^+ClO_4$

a) Première méthode

A 0,85 millimole de complexe Rh(Norbornadiène)(Acétylacétonate) et à 1 millimole de ligand L dissous dans 3 cm$^3$ de tétrahydrofuranne on ajoute, sous azote et à 20 °C, une solution de 120 mg d'acide perchlorique 70% dans 2 cm$^3$ de tétrahydrofuranne. Dès que la couleur de la solution devient orange, des cristaux apparaissent. Le phénomène s'amplifie par addition d'éther éthylique. On filtre sous azote, on lave au méthanol puis à l'éthanol et on sèche sous vide. On récupère ainsi le complexe ionique $[(Rh(Norbornadiène)L]^+ClO_4^-$ avec un rendement supérieur à 80%.

b) Deuxième méthode

A 1 millimole de complexes $Rh_2$(Norbornadiène)$_2Cl_2$ dissous dans 50 cm$^3$ de méthanol contenant 1 millimole de ligand L on ajoute, sous azote et à 20 °C, 1,1 millimole de perchlorate d'argent. Après 15 minutes d'agitation, on filtre le précipité de chlorure d'argent. Le filtrat est évaporé, lavé au benzène sous azote, puis à l'éther éthylique puis séché sous vide. Les cristaux du complexe ionique $[Rh(Norbornadiène)L]^+ClO_4^-$ sont ainsi récupérés avec un rendement supérieur à 80%.

Les spectre de résonance magnétique nucléaire du phosphore 31 pour différents complexes ainsi préparés permet leur identification selon la nature du ligand L (voir tableau IV).

EXEMPLES 17 à 19 – hydrogénation asymétrique

0,1 millimole d'un complexe ionique préparé conformément à l'exemple 24, dissous dans 10 cm$^3$ d'éthanol à 95%, est transféré sous hydrogène dans un réacteur préalablement purgé contenant 0,01 mole d'acide acétamidoacrylique dissous dans 40 cm$^3$ d'éthanol à 95%. On introduit de

l'hydrogène dans le réacteur et la réaction, effectuée à 20 °C sous pression atmosphérique, est suivie par lecture du volume d'hydrogène absorbé. Après 1 heure de réaction, l'éthanol est évaporé, le résidu est repris à l'eau chaude puis on filtre pour séparer le complexe insoluble. L'eau est évaporée et on récupère alors, avec un rendement chimique de 98%, la N-acétylalanine. Son pouvoir rotatoire spécifique $[\alpha]_D^{25}$, mesuré sur une solution aqueuse de concentration 2 g/100 $cm^3$, correspond au rendement optique R.O indiqué dans le tableau V ci-après en fonction de la nature du ligand L.

Tableau V

| Exemple | Ligand n° | $[\alpha]_D^{25}$ | R.O (%) |
|---|---|---|---|
| 17 | 2 | 31,7 | 47,7 |
| 18 | 7 | 41,8 | 62,8 |
| 19 | 5 | 53,2 | 80,0 |

EXEMPLE 20

On répète les opérations décrites aux exemples 17 à 19 en utilisant de l'hydrogène de haute pureté et en remplaçant l'éthanol à 95% par de l'éthanol pur, L étant le ligand n° 3. On récupère alors, avec le même rendement chimique, la N-acétylalamine dont le pouvoir rotatoire spécifique $[\alpha]_D^{25} = +$ 56,6% correspond à un rendement optique de 85%.

EXEMPLE 21

On répète les opérations décrites à l'exemple 19 en remplaçant l'acide acétamidoacrylique par l'acide acétamidocinnamique. On récupère alors la (S)N-acétylphénylalamine avec un rendement optique de 82%.

**Revendications**

1. Ligands phosphorés chiraux comprenant au moins un radical amine et au moins un radical dihydrocarbylphosphinoxy de formule W = $OP(R)_2$ dans laquelle R est un radical hydrocarboné choisi parmi les radicaux alkyles, aryles et cycloalkyles, caractérisés en ce qu'ils sont choisis parmi les aminophosphinites de formule

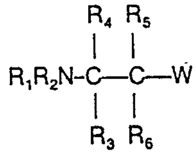

(I)

dans laquelle

- $R_1$ et $R_2$ sont choisis parmi l'atome hydrogène et les radicaux hydrocarbonés,
- $R_3$ et $R_4$, nécessairement différents, sont choisis parmi l'atome d'hydrogène et les radicaux hydrocarbonés éventuellement porteurs d'au moins une fonction choisie parmi les fonctions alcool, thiol, thiother, amine, imine, acide, ester, amide et ether,
- $R_5$ et $R_6$ sont choisis parmi l'atome d'hydrogène et les radicaux hydrocarbonés éventuellement fonctionnalisés.

2. Ligand phosphoré chiral selon la revendication 1, caractérisé en ce qu'il est choisi parmi:
- le (1R,2S)phényl-1-(oxydiphénylphosphino)-1-(N-méthylamino)-2-méthyl-2-éthane,
- le (S)(oxydiphénylphosphino)-1(N-méthylamino)-2-isopropyl-2-éthane,
- le (S)(oxyméthylene diphénylphosphino)-2-pyrrolidine, et
- le (S)(oxydiphénylphosphino)-1-(N-méthylamino)-2-éthane.

3. Ligand phosphoré chiral comprenant au moins un radical amine et au moins un radical dihydrocarbylphosphinoxy, caractérisé en ce qu'il est choisi parmi
- le (1R,2S) phényl-1-(oxydiphénylphosphino)-1-(N-méthyl-N-diphénylphosphinoamino)-2-méthyl-2-éthane,
- le (1R,2R) phényl-1-(oxydiphénylphosphino)-1-(N-méthyl-N-diphénylphosphinoamino)-2-méthyl-2-éthane,
- le (2S,4R)-N-diphénylphosphino-carbethoxy-2-(oxydiphényl-phosphino)4-pyrrolidine,
- le N-dicyclohexylphosphino-(oxyméthylène dicyclohexylphosphino)-2-pyrrolidine,
- le (S)N-diphénylphosphino-(oxyméthylène diphénylphosphino)-2-pyrrolidine,
- le (S)(oxydiphénylphosphino)-1-(N-méthyl-N-diphénylphosphino-amino)-2-phényl-2-éthane,
- le (S)(oxydiphénylphosphino)-1-(N-méthyl-N-diphénylphosphino-amino)-2-méthyl-2-éthane,
- le (S)(oxydiphénylphosphino)-1-(N-méthyl-N-diphénylphosphino-amino)-2-isopropyl-2-éthane,
- le (S)(oxydiphénylphosphino)-1-(N-méthyl-N-diphénylphosphino-amino)-2-isobutyl-2-éthane,
- le (S)(oxydiphénylphosphino)-1-(N-méthyl-N-diphénylphosphinoamino)-2-benzyl-2-éthane,
- le (S)-bis(oxydiphénylphosphino)-1,5-(N-méthyl-N-diphénylphosphinoamino)-2-pentane,
- le (S)-bis(oxydiphénylphosphino)-1,4-(N-méthyl-N-diphénylphosphinoamino)-2-butane,
- le (2S,3R)-bis(oxydiphénylphosphino)-1,3-(N-méthyl-N-diphénylphosphinoamino)-2-butane, et
- le (S)N,N'-bisdiphénylphosphino-(méthylamino)-2-(oxydiphénylphosphino)-1-(3-indolyl)-3-propane.

4. Procédé de fabrication de ligands phosphorés chiraux selon l'une des revendications 1 à 3, caractérisé en ce qu'il consiste à faire réagir dans un solvant hydrocarboné, à une température comprise entre −50 °C et 80 °C et sous atmosphère de gaz inerte un aminoalcool optiquement actif de formule générale

(V)

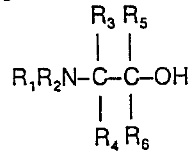

avec au moins un composé de formule $P(R)_2$ Y dans laquelle Y est choisi parmi les atomes d'halogène et les radicaux amines, ledit composé étant dans un rapport molaire, relativement à l'aminoalcool, supérieur ou égal au nombre de groupes $P(R)_2$ à introduire dans la molécule chirale.

5. Procédé selon la revendication 4 pour la fabrication de ligands phosphorés chiraux de formule (I) (II), caractérisé en ce que Y est un radical amine.

6. Procédé selon la revendication 4 pour la fabrication de ligands phosphorés chiraux de formule (III) ou (IV), caractérisé en ce que Y est un atome d'halogène.

7. Procédé selon la revendication 6, caractérisé en ce que la réaction est effectuée en présence d'un excès d'amine tertiaire.

8. Procédé selon l'une des revendications 4 à 7, caractérisé en ce que l'aminoalcool optiquement actif est choisi parmi le prolinol, l'hydroxyproline, les éphédrines et les aminoalcools N-méthylés dérivés d'aminoacides naturels choisis parmi glycine, phénylglycine, phénylalanine, leucine, sérine, théonine, histidine, lysine, arginine, isoleucine, valine, alanine, tryosine, tryptophane, méthionine, cystéine, glutamine, asparagine, acide aspartique, acide glytamique et cystine.

9. Application de ligands phosphorés chiraux selon l'une des revendications 1 à 3 à une réaction de synthèse énantiosélective de composés organiques, caractérisée en ce qu'elle consiste à faire réagir au moins un composé organique ne possédant pas de centre d'asymétrie avec (A) d'une part au moins un complexe de métal de transition de formule MZq dans laquelle M est un métal du groupe VIII de la Classification Périodique, q est le degré de coordination du métal M et Z est un atome ou une molécule capable de complexer le métal M et (B) d'autre part au moins un tel ligand phosphoré chiral.

10. Application d'une aminophosphine-phosphinite, ligand chiral de formule

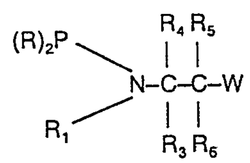

dans laquelle:

– R est un radical hydrocarboné choisi parmi les radicaux alkyles, aryles et cycloalkyles,

– $R_1$ est choisi parmi l'atome hydrogène et les radicaux hydrocarbonés,

– $R_3$ et $R_4$, nécessairement différents l'un de l'autre, sont choisis parmi l'atome d'hydrogène et les radicaux hydrocarbonés éventuellement porteurs d'au moins une fonction choisie parmi les fonctions alcool, thiol, thiother, amine, imine, acide, ester, amide et ether, et

– $R_5$ et $R_6$ sont choisis parmi l'atome d'hydrogène et les radicaux hydrocarbonés éventuellement fonctionnalisés,

à une réaction de synthèse énantiosélective de composés organiques, par réaction d'au moins un composé organique ne possédant pas de centre d'asymétrie avec (A) d'une part au moins un complexe de métal de transition de formule MZq dans laquelle M est un métal du groupe VIII de la Classification Périodique, q est le degré de coordination du métal M et Z est un atome ou une molécule capable de complexer le métal M et (B) d'autre part au moins un tel ligand chiral, caractérisée en ce que ladite réaction de synthèse énantiosélective est choisie parmi la réaction d'hydroformylation de substrats organiques insaturés à une température comprise entre 15 et 300 °C et sous une pression comprise entre 1 et 350 bars, la réaction d'hydrosilylation de cétones ou d'imines et les réactions de cyclodimérisation et dimérisation de diènes conjugués et de codimérisation de l'éthylène et d'un diène conjugué à une température comprise entre −30 et +100 °C et sous une pression inférieure à 100 bars.

11. Application selon l'une des revendications 9 et 10, caractérisée en ce que la réaction est effectuée en présence de (C) au moins un agent capable de capter au moins un ligand Z du constituant (A).

12. Application selon la revendication 11, caractérisée en ce que ledit agent est un acide possédant un antion $A^-$ peu coordinant et stériquement encombré ou un sel métallique d'un tel acide.

13. Application selon la revendication 11, caractérisée en ce que ledit agent est une quantité d'électricité mise en jeu par électrolyse à potentiel cathodique imposé.

14. Application selon la revendication 12, caractérisée en ce que l'anion $A^-$ est choisi parmi les anions perchlorate, tétrafluoroborate, tétraphénylborate et hexafluorophosphate.

15. Application selon l'une des revendications 12 et 14, caractérisée en ce que ledit sel métallique est un sel d'argent ou de thallium.

16. Application selon l'une des revendications 9 à 15, caractérisée en ce que la réaction est effectuée en présence de (D) au moins un activateur choisi parmi les dérivés aluminiques de formule $AlR_nX_{3-n}$, dans laquelle $0 \leqslant n \leqslant 3$, X est un atome d'halogène et R est un radical alkyle ayant de 1 à 12 atomes de carbone.

17. Application selon l'une des revendications 9 à 16, caractérisée en ce que le rapport molaire (B)/(A) est compris entre 1 et 10.

18. Application selon la revendication 12, caractérisée en ce que le rapport molaire (C)/(A) est inférieur ou égal à q.

19. Application selon l'une des revendications 16 à 18, caractérisée en ce que le rapport molaire (D)/(A) est compris entre 0,1 et 10.

20. Application selon l'une des revendications 9 à 19, caractérisée en ce que Z est choisi parmi le monoxyde de carbone, les halogènes, l'éthylène, le norbornadiène, le cyclooctadiène, et l'acétylacétone.

21. Application selon l'une des revendications 9 à 20, caractérisée en ce que le degré de coordination q est compris entre 2 et 6 inclusivement.

22. Application selon l'une des revendications 9 à 21, caractérisée en ce que la réaction de synthèse énantiosélective est une réaction d'hydrogénation de substrats organiques insaturés effectuée à une température comprise entre 0 et 200 °C et sous une pression comprise entre 1 et 200 bars.

23. Application selon l'une des revendications 9 à 21, caractérisée en ce que la réaction de synthèse énantiosélective est une réaction d'hydroformylation de substrats organiques insaturés effectuée à une température comprise entre 15 et 300 °C et sous une pression comprise entre 1 et 350 bars.

24. Application selon l'une des revendications 9 à 21, caractérisée en ce que la réaction de synthèse énantiosélective est une réaction d'hydrosilylation de cétones ou d'imines.

25. Application selon l'une des revendications 9 à 21, caractérisée en ce que la réaction de synthèse énantiosélective est choisie parmi les réactions de cyclodimérisation et dimérisation de diènes conjugués et les réactions de codimérisation de l'éthylène et d'un diène conjugué, et est effectuée à une température comprise entre −30 et +100 °C et sous une pression inférieure à 100 bars.

26. Complexes de métaux de transition utilisés comme intermédiaires dans une application selon l'une des revendications 9 à 25, caractérisés en ce qu'ils sont choisis parmi d'une part ceux de formule $MZ_{q-r}L_r$, et d'autre part ceux de formule $(MZ_{q-r}L_r)^+A^-$, formules dans lesquelles r est égal à 1 ou 2 et L désigne le ligand phosphoré chiral.

## Claims

1. Chiral phosphorous ligands comprising at least one amine radical and at least one dihydrocarbylphosphinoxy radical of formula W = $OP(R)_2$ in which R is a hydrocarbon radical chosen from alkyl, aryl and cycloalkyl radicals, which are characterized in that they are chosen from aminophosphinites of formula

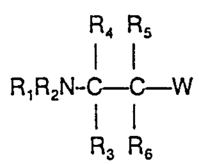

(I)

in which

– $R_1$ and $R_2$ are chosen from the hydrogen atom and hydrocarbon radicals,

– $R_3$ and $R_4$, which are necessarily different, are chosen from the hydrogen atom and hydrocarbon radicals optionally carrying at least one functional group chosen from the alcohol, thiol, thoether, amine, imine, acid, ester, amide and ether functional groups,

– $R_5$ and $R_6$ are chosen from the hydrogen atom and hydrocarbon radicals optionally containing functional groups.

2. Chiral phosphorous ligand according to Claim 1, characterized in that it is chosen from:
- (1R,2S)-1-phenyl-1-(oxydiphenylphosphino)-2-(N-methylamino)-2-methylethane,
- (S)-1-(oxydiphenylphosphino)-2-(N-methylamino)-2-isopropylethane,
- (S)-2-(oxymethylenediphenylphosphino)pyrrolidine, and
- (S)-1-(oxydiphenylphosphino)-2-(N-methylamino)ethane.

3. Chiral phosphorous ligand comprising at least one amine radical and at least one dihydrocarbylphosphinoxy radical, characterized in that it is chosen from
- (1R,2S)-1-phenyl-1-(oxydiphenylphosphino)-2-(N-methyl-N-diphenylphosphinoamino)-2-methylethane,
- (1R,2R)-1-phenyl-1-(oxydiphenylphosphino)-2-(N-methyl-N-diphenylphosphinoamino)-2-methylethane,
- (2S,4R)-N-diphenylphosphino-2-carbethoxy-4-(oxydiphenylphosphino)pyrrolidine,
- N-dicyclohexylphosphino-2-(oxymethylenephosphino)-pyrrolidine,
- (S)-N-diphenylphosphino-2-(oxymethylenediphenylphosphino)pyrrolidine,
- (S)-1-(oxydiphenylphosphino)-2-(N-methyl-N-diphenylphosphinoamino)-2-phenylethane,
- (S)-1-(oxydiphenylphosphino)-2-(N-methyl-N-diphenylphosphinoamino)-2-methylethane,
- (S)-1-(oxydiphenylphosphino)-2-(N-methyl-N-diphenylphosphinoamino)-2-isopropylethane,
- (S)-1-(oxydiphenylphosphino)-2-(N-methyl-N-diphenylphosphinoamino)-2-isobutylethane,
- (S)-1-(oxydiphenylphosphino)-2-(N-methyl-N-diphenylphosphinoamino)-2-benzylethane,
- (S)-1,5-bis(oxydiphenylphosphino)-2-(N-methyl-N-diphenylphosphinoamino)pentane,
- (S)-1,4-bis(oxydiphenylphosphino)-2-(N-methyl-N-diphenylphosphinoamino)butane,
- (2S,3R)-1,3-bis(oxydiphenylphosphino)-2-(N-methyl-N-diphenylphosphinoamino)butane, and
- (S)-N,N'-bisdiphenylphosphino-2-(methylamino)-1-(oxydiphenylphosphino)-3-(3-indolyl)propane.

4. Process for the manufacture of chiral phosphorous ligands according to one of Claims 1 to 3, characterized in that it consists in reacting an optically active aminoalcohol of general formula

(V)

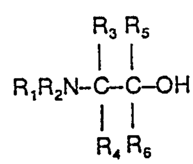

in a hydrocarbon solvent, at a temperature of between −50 °C and 80 °C and under an inert gas atmosphere, with at least one compound of formula $P(R)_2Y$ in which Y is chosen from halogen atoms and amine radicals, the said compound being in a molar ratio, relative to the aminoalcohol, which is higher than or equal to the number of $P(R)_2$ groups to be introduced into the chiral molecule.

5. Process according to Claim 4 for the manufacture of chiral phosphorous ligands of formula (I) (II), characterized in that Y is an amine radical.

6. Process according to Claim 4 for the manufacture of chiral phosphorous ligands of formula (III) or (IV), characterized in that Y is a halogen atom.

7. Process according to Claim 6, characterized in that the reaction is carried out in the presence of an excess of tertiary amine.

8. Process according to one of Claims 4 to 7, characterized in that the optically active aminoalcohol is chosen from prolinol, hydroxyproline, ephedrines and N-methylated aminoalcohols derived from natural amino acids chosen from glycine, phenylglycine, phenylalanine, leucine, serine, threonine, histidine, lysine, arginine, isoleucine, valine, alanine, tyrosine, tryptophan, methionine, cysteine, glutamine, asparagine, aspartic acid, glutamic acid and cystine.

9. Application of chiral phosphorous ligands according to one of Claims 1 to 3 to an enantioselective synthesis reaction of organic compounds, characterized in that it consists in reacting at least one organic compound containing no centre of asymmetry with (A) on the one hand at least one transition metal complex of formula MZq in which M is a metal of group VIII of the Periodic Classification, q is the coordination number of the metal M and Z is an atom or a molecule capable of complexing the metal M and (B) on the other hand at least one chiral phosphorous ligand of this kind.

10. Application of an aminophosphinephosphinite, a chiral ligand of formula

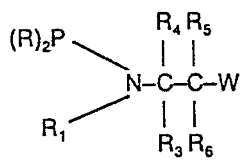

in which:

– R is a hydrocarbon radical chosen from alkyl, aryl and cycloalkyl radicals,

– $R_1$ is chosen from the hydrogen atom and hydrocarbon radicals,

– $R_3$ and $R_4$, which are necessarily different from each other, are chosen from the hydrogen atom and hydrocarbon radicals optionally carrying at least one functional group chosen from the alcohol, thio, thioether, amine, imine, acid, ester, amide and ether functional groups, and

– $R_5$ and $R_6$ are chosen from the hydrogen atom and hydrocarbon radicals optionally containing functional groups, to an enantioselective synthesis reaction of organic compounds, by reaction of at least one compound containing no centre of asymmetry with (A) on the one hand at least one transition metal complex of formula MZq in which M is a metal of group VIII of the Periodic Classification, q is the coordination number of the metal M and Z is an atom or a molecule capable of complexing the metal M and (B) on the other hand at least one chiral ligand of this kind, characterized in that the said enantioselective synthesis reaction is chosen from the reaction of hydroformylation of unsaturated organic substrates at a temperature of between 15 and 300 °C and at a pressure of between 1 and 350 bars, the reaction of hydrosilylation of ketones or of imines and the reactions of cyclodimerization and dimerization of conjugated dienes and of codimerization of ethylene and of a conjugated diene at a temperature of between −30 and +100 °C and at a pressure below 100 bars.

11. Application according to either of Claims 9 and 10, characterized in that the reaction is carried out in the presence of (C) at least one agent capable of capturing at least one ligand Z of the constituent (A).

12. Application according to Claim 11, characterized in that the said agent is an acid which has a relatively weakly coordinating and sterically hindered anion $A^-$ or a metal salt of an acid of this kind.

13. Application according to Claim 11, characterized in that the said agent is a quantity of electricity employed by means of electrolysis at an imposed cathode potential.

14. Application according to Claim 12, characterized in that the anion $A^-$ is chosen from the perchlorate, tetrafluoroborate, tetraphenylborate and hexafluorophosphate anions.

15. Application according to either of Claims 12 and 14, characterized in that the said metal salt is a silver or thallium salt.

16. Application according to one of Claims 9 to 15, characterized in that the reaction is carried out in the presence of (D) at least one activator chosen from the aluminium derivatives of formula $AlR_nX_{3-n}$, in which $0 \leq n \leq 3$, X is a halogen atom and R is an alkyl radical containing from 1 to 12 carbon atoms.

17. Application according to one of Claims 9 to 16, characterized in that the molar ratio (B)/(A) is between 1 and 10.

18. Application according to Claim 12, characterized in that the molar ratio (C)/(A) is lower than or equal to q.

19. Application according to one of Claims 16 to 18, characterized in that the molar ratio (D)/(A) is between 0.1 and 10.

20. Application according to one of Claims 9 to 19, characterized in that Z is chosen from carbon monoxide, the halogens, ethylene, norbornadiene, cyclooctadiene and acetylacetone.

21. Application according to one of Claims 9 to 20, characterized in that the coordination number q is between 2 and 6 inclusive.

22. Application according to one of Claims 9 to 21, characterized in that the enantioselective synthesis reaction is a reaction of hydrogenation of unsaturated organic substrates which is carried out at a temperature of between 0 and 200 °C and at a pressure of between 1 and 200 bars.

23. Application according to one of Claims 9 to 21, characterized in that the enantioselective synthesis reaction is a reaction of hydroformylation of unsaturated organic substrates which is carried out at a temperature of between 15 and 300 °C and at a pressure of between 1 and 350 bars.

24. Application according to one of Claims 9 to 21, characterized in that the enantioselective synthesis reaction is a reaction of hydrosilylation of ketones or of imines.

25. Application according to one of Claims 9 to 21, characterized in that the enantioselective synthesis reaction is chosen from the reactions of

cyclodimerization and dimerization of conjugated dienes and the reactions of codimerization of ethylene and of a conjugated diene, and is carried out at a temperature of between −30 and +100 °C and at a pressure below 100 bars.

26. Transition metal complexes employed as intermediates in an application according to one of Claims 9 to 25, characterized in that they are chosen from, on the one hand, those of formula $MZ_{q\text{-}r}L_r$ and, on the other hand, those of formula $(MZ_{q\text{-}r}L_r)^+A^-$, in which formulae r is equal to 1 or 2 and L denotes the chiral phosphorous ligand.

**Patentansprüche**

1. Chirale Phosphor-Liganden enthaltend mindestens ein Aminradikal und mindestens ein Dihydrocarbylphosphinoxyradikal der Formel W = $OP(R)_2$, in der R ein aus den Alkyl-, Aryl- und Cycloalkylradikalen ausgewähltes Kohlenwasserstoffradikal ist, dadurch gekennzeichnet, daß sie ausgewählt sind aus den Aminophosphiniten der Formel

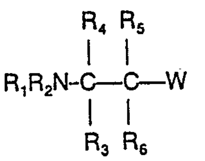

in der (I)

– $R_1$ und $R_2$ ausgewählt sind aus der Gruppe bestehend aus dem Wasserstoffatom und den Kohlenwasserstoffradikalen,

– $R_3$ und $R_4$, die notwendigerweise verschieden sind, ausgewählt sind aus der Gruppe bestehend aus dem Wasserstoffatom und den Kohlenwasserstoffradikalen, die gegebenenfalls Träger mindestens einer Funktion sind, die ausgewählt ist aus der Gruppe bestehend aus den Alkohol-, Thiol-, Thioether-, Amin-, Imin-, Säure-, Ester-, Amid- und Etherfunktionen, und

– $R_5$ und $R_6$ ausgewählt sind aus der Gruppe bestehend aus dem Wasserstoffatom und den gegebenenfalls funktionalisierten Kohlenwasserstoffradikalen.

2. Chiraler Phosphor-Ligand nach Anspruch 1, dadurch gekennzeichnet, daß er ausgewählt ist aus der Gruppe bestehend aus:
- (1R,2S)Phenyl-1-(oxydiphenylphoshino)-1-(N-methylamino)-2-methyl-2-ethan,
- (S)(Oxydiphenylphosphino)-1-(N-methylamino)-2-isopropyl-2-ethan,
- (S)(Oxymethylen diphenylphosphino)-2-pyrrolidin, und
- (S)(Oxydiphenylphosphino)-1-(N-methylamino)-2-ethan.

3. Chiraler Phosphor-Ligand enthaltend mindestens ein Aminradikal und mindestens ein Dihydrocarbylphosphinoxyradikal, dadurch gekennzeichnet, daß er ausgewählt ist aus der Gruppe bestehend aus:
- (1R,2S) Phenyl-1-(oxydiphenylphosphino)-1-(N-methyl-N-diphenylphosphinamino)-2-methyl-2-ethan,
- (1R,2R) Phenyl-1-(oxydiphenylphosphino)-1-(N-methyl-N-diphenylphosphinamino)-2-methyl-2-ethan,
- (2S,4R)-N-Diphenylphosphino-carbethoxy-2-(oxydiphenyl-phosphino)4-pyrrolidin,
- N-Dicyclohexylphosphino-(oxymethylen dicyclohexylphosphino)-2-pyrrolidin,
- (S)(N-Diphenylphosphino-(oxymethylen diphenylphosphino)-2-pyrrolidin,
- (S)(Oxydiphenylphosphino)-1-(N-methyl-N-diphenylphosphinamino)-2-phenyl-2-ethan,
- (S)(Oxydiphenylphosphino)-1-(N-methyl-N-diphenylphosphinamino)-2-methyl-2-ethan,
- (S)(Oxydiphenylphosphino)-1-(N-methyl-N-diphenylphosphinamino)-2-isopropyl-2-ethan,
- (S)(Oxydiphenylphosphino)-1-(N-methyl-N-diphenylphosphinamino)-2-isobutyl-2-ethan,
- (S)(Oxydiphenylphosphino)-1-(N-methyl-N-diphenylphosphinamino)-2-benzyl-2-ethan,
- (S)-bis(Oxydiphenylphosphino)-1,5-(N-methyl-N-diphenylphosphinamino)-2-pentan,
- (S)-bis(Oxydiphenylphosphino)-1,4-(N-methyl-N-diphenylphosphinamino)-2-butan,
- (2S,3R)-bis(Oxydiphenylphosphino)-1,3-(N-methyl-N-diphenylphosphinamino)-2-butan, und
- (S)N,N′-Bisdiphenylphosphino-(methylamino)-2-(oxydiphenylphosphino)-1-(3-indolyl)-3-propan.

4. Verfahren zur Herstellung von chiralen Phosphor-Liganden nach einem der Ansprüche 1 bis 3, dadurch gekennzeichnet, daß es darin besteht, daß in einem Kohlenwasserstofflösungsmittel bei einer Temperatur zwischen −50° und 80 °C und unter Inertgasatmosphäre ein optisch aktiver Aminoalkohol der allgemeinen Formel

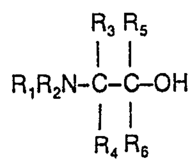

(V)

mit mindestens einer Verbindung der Formel $P(R)_2$ Y zur Reaktion gebracht wird, in welcher Formel Y ausgewählt ist aus der Gruppe bestehend aus den Halogenatomen und den Aminradikalen, wobei die genannte Verbindung in einem Molverhältnis in bezug auf den Aminoalkohol vorliegt, das größer als die oder gleich der Anzahl der in das chirale Molekül einzubringenden $P(R)_2$-Gruppen ist.

5. Verfahren nach Anspruch 4 zur Herstellung von chiralen Phosphor-Liganden der Formel (I) oder (II), dadurch gekennzeichnet, daß Y ein Aminradikal ist.

6. Verfahren nach Anspruch 4 zur Herstellung von chiralen Phosphor-Liganden der Formel (III) oder (IV), dadurch gekennzeichnet, daß Y ein Halogenatom ist.

7. Verfahren nach Anspruch 6, dadurch gekennzeichnet, daß die Reaktion in Gegenwart eines Überschusses an tertiärem Amin durchgeführt wird.

8. Verfahren nach einem der Ansprüche 4 bis 7, dadurch gekennzeichnet, daß der optisch aktive Aminoalkohol ausgewählt ist aus der Gruppe bestehend aus Prolinol, Hydroxyprolin, den Ephedrinen und den N-methylierten Alkoholen, welche von natürlichen Aminosäuren abgeleitet sind, die ausgewählt sind aus der Gruppe bestehend aus Glycin, Phenylglycin, Phenylalanin, Leucin, Serin, Theonin, Histidin, Lysin, Arginin, Isoleucin, Valin, Alanin, Tyrosin, Tryptophan, Methionin, Cystein, Glutamin, Asparagin, Asparaginsäure, Glutaminsäure und Cystin.

9. Anwendung von chiralen Phosphor-Liganden nach einem der Ansprüche 1 bis 3 bei einer enantioselektiven Synthese-Reaktion organischer Verbindungen, dadurch gekennzeichnet, daß sie darin besteht, daß mindestens eine organische Verbindung, die kein Asymmetriezentrum besitzt, einerseits mit (A) mindestens einem Übergangsmetallkomplex der Formel MZq, in der M ein Metall der Gruppe VIII des Periodensystems, q die Koordinationszahl des Metalls M und Z ein Atom oder ein Molekül ist, das zur Komplexbildung mit dem Metall M fähig ist, und anderseits mit (B) einem solchen chiralen Phosphor-Liganden zur Reaktion gebracht wird.

10. Anwendung eines chiralen Aminophosphinphosphinit-Liganden der Formel

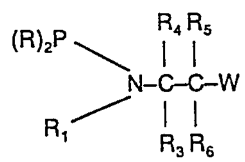

in der:

– R ein aus den Alkyl-, Aryl- und Cycloalkylradikalen ausgewähltes Kohlenwasserstoffradikal ist,

– $R_1$ ausgewählt ist aus der Gruppe bestehend aus dem Wasserstoffatom und den Kohlenwasserstoffradikalen,

– $R_3$ und $R_4$, die notwendigerweise verschieden sind, ausgewählt sind aus der Gruppe bestehend aus dem Wasserstoffatom und den Kohlenwasserstoffradikalen, die gegebenenfalls Träger mindestens einer Funktion sind, die ausgewählt ist aus der Gruppe bestehend aus den Alkohol-, Thiol-, Thioether-, Amin-, Imin-, Säure-, Ester-, Amid- und Etherfunktionen, und

– $R_5$ und $R_6$ ausgewählt sind aus der Gruppe bestehend aus dem Wasserstoffatom und den gegebenenfalls funktionalisierten Kohlenwasserstoffradikalen, bei einer enantioselektiven Synthese-Reaktion organischer Verbindungen durch Reaktion mindestens einer kein Asymmetriezentrum besitzenden organischen Verbindung einerseits mit (A) mindestens einem Übergangsmetallkomplex der Formel MZq, in der M ein Metall der Gruppe VIII des Periodensystems, q die Koordinationszahl des Metalls M und Z ein Atom oder ein Molekül ist, das zur Komplexbildung mit dem Metall M fähig ist, und anderseits mit (B) mindestens einem solchen chiralen Liganden, dadurch gekennzeichnet, daß die genannte enantioselektive Synthese-Reaktion ausgewählt ist aus der Gruppe bestehend aus der Hydroformylierungsreaktion ungesättigter organischer Substrate bei einer Temperatur zwischen 15° und 300 °C und unter einem Druck zwischen 1 und 350 bar, die Hydrosilylierungsreaktion von Ketonen oder Iminen und die Cyclodimerisations- und Dimerisationsreaktionen konjugierter Diene sowie die Codimerisationsreaktion des Ethylens und eines konjugierten Diens bei einer Temperatur zwischen −30° und +100 °C und unter einem Druck unter 100 bar.

11. Anwendung nach Anspruch 9 oder 10, dadurch gekennzeichnet, daß die Reaktion in Gegenwart (C) mindestens eines Mittels erfolgt, das fähig ist, mindestens einen Liganden Z des Bestandteils (A) zu binden.

12. Anwendung nach Anspruch 11, dadurch gekennzeichnet, daß das genannte Mittel eine Säure, die ein wenig koordinierendes und sterisch gehindertes Anion $A^-$ besitzt, oder ein Metallsalz einer solchen Säure ist.

13. Anwendung nach Anspruch 11, dadurch gekennzeichnet, daß das genannte Mittel eine durch Elektrolyse mit angelegtem Kathodenpotential ins Spiel gebrachte Elektrizitätsmenge ist.

14. Anwendung nach Anspruch 12, dadurch gekennzeichnet, daß das Anion $A^-$ ausgewählt ist aus der Gruppe bestehend aus den Perchlorat-, den Tetrafluorborat-, den Tetraphenylborat- und den Hexafluorphosphatanionen.

15. Anwendung nach Anspruch 12 oder 14, dadurch gekennzeichnet, daß das genannte Metallsalz ein Silber- oder Thalliumsalz ist.

16. Anwendung nach einem der Ansprüche 9 bis 15, dadurch gekennzeichnet, daß die Reaktion in Gegenwart (D) mindestens eines Aktivators erfolgt, der ausgewählt ist aus den Aluminiumverbindungen der Formel $AlR_nX_{3-n}$, in der $0 \leq n \leq 3$, X ein Halogenatom und R ein Alkylradikal mit 1 bis 12 Kohlenstoffatomen ist.

17. Anwendung nach einem der Ansprüche 9 bis 16, dadurch gekennzeichnet, daß das Molverhältnis (B)/(A) zwischen 1 und 10 beträgt.

18. Anwendung nach Anspruch 12, dadurch gekennzeichnet, daß das Molverhältnis (C)/(A) geringer als oder gleich q ist.

19. Anwendung nach einem der Ansprüche 16 bis 18, dadurch gekennzeichnet, daß das Molverhältnis (D)/(A) zwischen 0,1 und 10 beträgt.

20. Anwendung nach einem der Ansprüche 9 bis 19, dadurch gekennzeichnet, daß Z ausgewählt ist aus der Gruppe bestehend aus Kohlenmonoxid, den Halogenen, Ethylen, Norbornadien, Cyclooctadien und Acetylaceton.

21. Anwendung nach einem der Ansprüche 1 bis 20, dadurch gekennzeichnet, daß die Koordinationszahl q 2 bis 6 beträgt.

22. Anwendung nach einem der Ansprüche 9 bis 21, dadurch gekennzeichnet, daß die enantioselektive Synthese-Reaktion eine Hydrierungsreaktion von ungesättigten organischen Substraten ist, welche bei einer Temperatur zwischen 0° und 200 °C und unter einem Druck zwischen 1 und 200 bar erfolgt.

23. Anwendung nach einem der Ansprüche 9 bis 21, dadurch gekennzeichnet, daß die enantioselektive Synthese-Reaktion eine Hydroformylierungsreaktion von ungesättigten organischen Substraten ist, welche bei einer Temperatur zwischen 15° und 300 °C und unter einem Druck zwischen 1 und 350 bar erfolgt.

24. Anwendung nach einem der Ansprüche 9 bis 21, dadurch gekennzeichnet, daß die enantioselektive Synthese-Reaktion eine Hydrosilylierungsreaktion von Ketonen oder Iminen ist.

25. Anwendung nach einem der Ansprüche 9 bis 21, dadurch gekennzeichnet, daß die enantioselektive Synthese-Reaktion ausgewählt ist aus der Gruppe bestehend aus den Cyclodimerisations- und Dimerisationsreaktionen konjugierter Diene und den Codimerisationsreaktionen des Ethylens und eines konjugierten Diens, und bei einer Temperatur zwischen $-30°$ und $+100\,°C$ und unter einem Druck von weniger als 100 bar erfolgt.

26. Übergangsmetallkomplexe, die bei einer Anwendung nach einem der Ansprüche 9 bis 25 als Zwischenprodukte eingesetzt werden, dadurch gekennzeichnet, daß sie ausgewählt sind einerseits aus jenen der Formel $MZ_{q-r}L_r$ und anderseits aus jenen der Formel $(MZ_{q-r}L_r)^+A^-$, in welchen Formeln $r = 1$ oder 2 und L den chiralen Phosphor-Liganden bezeichnet.